# EUROPEAN PATENT APPLICATION

(11) **EP 4 616 843 A1**
(43) Date of publication of application: **17.09.2025**
(21) Application number: 24163340.3
(22) Date of filing: 13.03.2024
(51) Int. Cl.: A61K 9/00, A61K 31/737, A61K 47/02, A61K 9/08

(54) **PHARMACEUTICAL COMPOSITION FOR USE IN THE TREATMENT OF ULCERATIVE COLITIS AND IN THE TREATMENT OF INTERSTITIAL CYSTITIS/BLADDER PAIN SYNDROME**

(71) Applicant: Mirkin, Iakov, 350000 Krasnodar (RU); Ponomarenko, Maksim, 350000 Krasnodar (RU)
(72) Inventor: Mirkin, Iakov, 350000 Krasnodar (RU); Ponomarenko, Maksim, 350000 Krasnodar (RU)
(74) Representative: Benatov, Samuil Gabriel

(57) **Abstract**

Present invention relates to a pharmaceutical composition, which is suitable for use in the prevention, treatment, or alleviation of ulcerative colitis, and in the prevention, treatment, or alleviation of Interstitial Cystitis/Bladder Pain Syndrome (IC/BPS). Invention relates also to a medical device comprising the pharmaceutical composition in a package with leaflet.

## Description

### TECHNIAL FIELD OF THE INVENTION

Present invention relates to a pharmaceutical composition, which is suitable for use in the prevention, treatment, or alleviation of ulcerative colitis, and in the prevention, treatment, or alleviation of Interstitial Cystitis/Bladder Pain Syndrome (IC/BPS). Invention relates also to a medical device comprising the pharmaceutical composition in a package with leaflet.

### BACKGROUND ART OF THE INVENTION

Inflammatory bowel diseases (IBD) are chronic and recurring diseases of the gastrointestinal tract that are characterized by abdominal pain, diarrhoea, rectal bleeding and weight loss. Ulcerative Colitis and Crohn's Disease are the two most common types of IBD, where patients either experience acute attacks lasting weeks to months followed by periods of remission or relapse, or chronic persistent inflammation. Choice of conventional IBD therapies depend on the severity of the disease and aim to relieve symptoms or induce and maintain remission in order to improve the quality of life of the patient [Danese and Fiocchi, 2011]. The treatment with anti-inflammatory drugs, such as salicylates e.g. sulfasalazine, balsalazide and olsalazine, which are prodrugs of 5-aminosalicylic acid (5-ASA), is usually the primary choice in mild-moderate courses of the disease [Travis et al., 2008], whereas more severe forms involve the use of steroids and immunosuppressive drugs. In such cases, 5-ASA preparations are administered as oral or rectal formulations and a combination of both formulations is considered to be more effective in mild to moderate colitis [Marteau et al., 2005]. However, in severe cases, 5-ASA often proves to be less effective. Glycosaminoglycans (GAGs) such as hyaluronic acid (HA) are biomacromolecules that are extensively present in the human body namely in biological fluids and connective tissues [Volpi et al., 2009]. HA is a linear, non-sulphated GAG consisting of disaccharide units made up of D-glucuronic acid and N-acetyl-D-glucosamine linked by glycosidic β-1,3 bond. IBD is a chronic disease in which pharmacotherapy typically requires continuous long-term drug therapy. Therefore, adverse effects of the anti-inflammatory drugs play an important role in maintaining the patient's quality of life. 5-ASA combined with HA is a promising drug combination, especially since 5-ASA is rather well-tolerated. However, its efficiency in severe cases of colitis can be limited and data suggest the combination of rectally administered HA and 5-ASA therapy might provide an increased benefit in the treatment of severe colitis [Biomol Ther 29(5), 536-544 (2021)]

Henusha D. Jhundoo et all conducted a study, wherein both 5-ASA (30 mg/kg) and HA (15 mg/kg or 30 mg/kg) were administered rectally and investigated for their potential complementary therapeutic effects in moderate or severe murine colitis models. Intrarectal treatment of moderate and severe colitis with 5-ASA alone or HA alone at a dose of 30 mg/kg led to a significant decrease in clinical activity and histology scores, myeloperoxidase activity (MPO), TNF-α, IL-6 and IL-1β in colitis mice compared to untreated animals. The combination of HA (30 mg/kg) and 5-ASA in severe colitis led to a significant improvement of colitis compared to 5-ASA alone.

### [Biomol Ther 29(5), 536-544 (2021)]

Interstitial Cystitis/Bladder Pain Syndrome (IC/BPS) is a chronic clinical syndrome characterized by a bladder/pelvic pain and urinary dysfunction, such as increased frequency and urgency, with a strongly negative impact on patients' quality of life. The etiology of IC/BPS is still not well understood, and different hypotheses have been formulated, including autoimmune processes, allergic reactions, chronic bacterial infections, exposure to toxins or dietary elements, and psychosomatic factors. It has been hypothesized that IC/BPS could be pathophysiologically related to a disruption of the bladder mucosa with consequent loss of glycosaminoglycans (GAGs), a class of mucopolysaccharides with hydrorepellent properties, exposing the urothelium to many urinary toxic agents. Once these substances penetrate into the bladder wall, a chain reaction is thought to be triggered in the submucosa, where nerve terminals produce in inflammatory mediators causing mast cell degranulation and histamine secretion with consequent vasodilatation and inflammatory exudate. This inflammatory response stimulates C fibers with consequent bladder pain and release of neuropeptides, producing damage to the mucosa and fibrosis of the submucosa. Based on these aspects, the early repair of the bladder mucosa with intravesical application of GAGs, such as hyaluronic acid (HA) or chondroitin sulphate (CS), has been proposed as a possible treatment. Hyaluronic acid is a naturally occurring proteoglycan present in the GAG layer of the bladder urothelium. It has been proposed that the intravesical application of HA can promote regeneration of the GAG, the deficit of which has been demonstrated in patients with IC/BPS and is considered to play an important role in the pathogenesis of the disease. Hyaluronic acid also, has an inhibitory action on mast cells degranulation, which its activation is a crucial step in the pathogenesis of IC/BPS. Chondroitin sulphate is another natural proteoglycan present in the GAG layer of the bladder epithelium. Like HA, intravesical instillation of CS has been used as a treatment for patients with IC/BPS, to help in the regeneration of GAG in the bladder urothelium. A previous study revealed that good control of urinary symptoms and pain was achieved with CS, suggesting that this drug may be useful in treatment of IC/BPS. Hyaluronic acid acts by increasing production of GAG from epithelial cells through activation of GAG enzymes that leads to increased GAG secretion, restoring normal GAG barrier production. HA also, acts by decreasing the permeability of the urothelium to urine constitutes. Chondroitin sulphate is another natural proteoglycan which is used to replenish the protective GAG layer and its usage alone without HA yielded low response rates [Turk J Urol. 2019 Jul; 45(4): 296-301.]

Mucoadhesive formulations are mainly intended for oral/buccal, gastroesophageal, nasal, or vaginal administration routes. They contain one or more substances/polymers of either natural, synthetic, or semi-synthetic origin endowed with mucoadhesive properties. These are complex substances whose chemical-physical properties are in general well characterized. Hydration and water retaining properties, gel formation, lubricating properties are example of functional characteristics that may be involved in mucosal interaction. Hyaluronic acid is a typical representative of mucoadhesive polymers.

The more general term bioadhesion commonly defines the adhesion of a substance (i.e., polymeric-based products, such as dosage forms or medical devices) to a biological tissue: the substance-biologic tissue adhesive interaction allows an intimate contact between the two materials for an extended period of time. The higher the strength of the adhesive interaction, the higher the residence time of the substance on the biologic tissue. When the biologic tissue is represented by the mucus layer that covers a mucosal tissue, the phenomenon is referred to as mucoadhesion [Edsman and Hägerström, 2005; Smart, 2005; Cook and Khutoryanskiy, 2015]. The mucoadhesive materials are typically high molecular weight macromolecules and as such they are not systemically absorbed. [Front. Drug. Saf. Regul. 3:1227763]

A product - mucoadhesive gel for gastro-esophageal reflux syndrome: ESOXX is known. The product, which is suitable for gastro-esophageal application, is a combination of hyaluronic acid, chondroitin sulphate, PVP, poloxamer 407, and contains xylitol C, sodium benzoate, potassium sorbate as preservative, flavouring agents, and purified water. The gel acts as a physical barrier, protecting the gastro-esophageal mucosa against the attach of gastric juice. As for the composition, it is noted that PVP is considered an adhesive substance in general as suggested by its chemical nature, whereas chondroitin sulphate mainly favours the reparative processes of the underlying mucosa. Instead, the combination of hyaluronic acid with poloxamer represents a solution since a synergic effect has been demonstrated between the two polymer types in promoting jellification and gel strengthening. [Front. Drug. Saf. Regul. 3:1227763]

A Russian patent RU2807235C1 discloses medicinal product "Urolife", which is a sterile viscoelastic protector for the bladder mucosa. It contains in each 1 ml: hyaluronic acid 0.8 mg, sodium dihydrophosphate monohydrate 0.45 mg, phosphate anhydrous 2 mg, sodium chloride 8.5 mg, deionised water.

The mucous membrane of the epithelium of the bladder and rectum is covered with the so-called glycosaminoglycan layer, which consists of Hyaluronic acid, Chondroitin sulphate, Dermatan sulphate, Heparan sulphate. The combination of these substances protects the epithelium from the penetration of aggressive substances from the cavity of the bladder or intestines into the deep layers of the epithelium, i.e. represents a protective barrier. A problem is when this barrier is destroyed or dysfunctional, substances and antigens from the intestinal lumen or bladder penetrate the epithelium and cause inflammation: in the bladder - interstitial cystitis; in the intestines - ulcerative colitis.

Treatment of ulcerative (interstitial) cystitis or ulcerative (nonspecific) colitis using instillations of the bladder or intestines with components of the protective (glycosaminoglycan) layer is called glycosaminoglycan replacement therapy. The problem with this therapy is that the clinical picture of ulcerative cystitis is characterized by frequent urination, and ulcerative colitis is characterized by frequent bowel movements. It is believed that the exposure of the drug in the intestines or bladder should range from 30 minutes to 2 hours or more. That is, with pronounced clinical manifestations of ulcerative cystitis or ulcerative colitis, the drug will leave the target organ before it has time to have a therapeutic effect.

The disadvantages of products for use in the prevention, treatment or alleviation of interstitial cystitis or ulcerative colitis are: lack of individualised treatment; high medication load; insufficient reduction of signs of inflammation of the bladder or colonic mucosa, determined by morphological examination of colonic or bladder mucosa biopsy at rectoscopy, colonoscopy or cystoscopy after 2 months of treatment; recurrences of the disease within a year; insufficiently rapid subsidence of clinical manifestations: diarrhoea, blood discharge during defecation, etc.

The object of present invention is to provide a pharmaceutical composition, which is suitable for use effectively and efficiently in the prevention, treatment, or alleviation of ulcerative colitis and in the prevention, treatment, or alleviation of Interstitial (ulcerative) Cystitis/Bladder Pain Syndrome (IC/BPS).

### SUMMARY OF THE INVENTION

In a first aspect, the object of the invention is achieved by providing a pharmaceutical composition comprising: hyaluronic acid, sodium dihydrophosphate monohydrate, sodium phosphate anhydrous, sodium chloride and deionised water, characterized in that it further comprises chondroitin sulphate and mucoadhesive biopolymer.

The pharmaceutical composition according to invention is intended for administration of a single application dose in the bladder or rectum via a urethral or rectal catheter, or rectal applicator. Composition has a protective effect on the bladder, rectal and colon mucosa and at the same time has a tissue repair and regeneration effect.

In one embodiment of the invention, the pharmaceutical composition is for use in the prevention, treatment, or alleviation of ulcerative colitis.

In another embodiment of the invention, the pharmaceutical composition is for use in the prevention, treatment, or alleviation of Interstitial (ulcerative) Cystitis/Bladder Pain Syndrome (IC/BPS).

In a further aspect, the present invention provides a medical device comprising the pharmaceutical composition in a package with leaflet.

The pharmaceutical composition can be put in packages as follows: glass bottle 50 ml, pre-filled syringe 50 ml, accordeon bottle 30 - 50 ml.

### BRIEF DESCRIPTION OF THE FIGURES

Further feature and advantages of the invention can be ascertained from the following figures described below:
Fig. 1: Survival of rats during the experiment (Kaplan-Meier)
Fig. 2: Disease Activity Index (DAI)
Fig. 3. Changes in calprotectin content in rat feces
Fig. 4: 1o - pronounced necrotic changes in the wall, diffuse neutrophilic infiltration of the mucosal and submucosal layers (proximal and distal parts - from left to right). - sample has been taken from control (saline) group

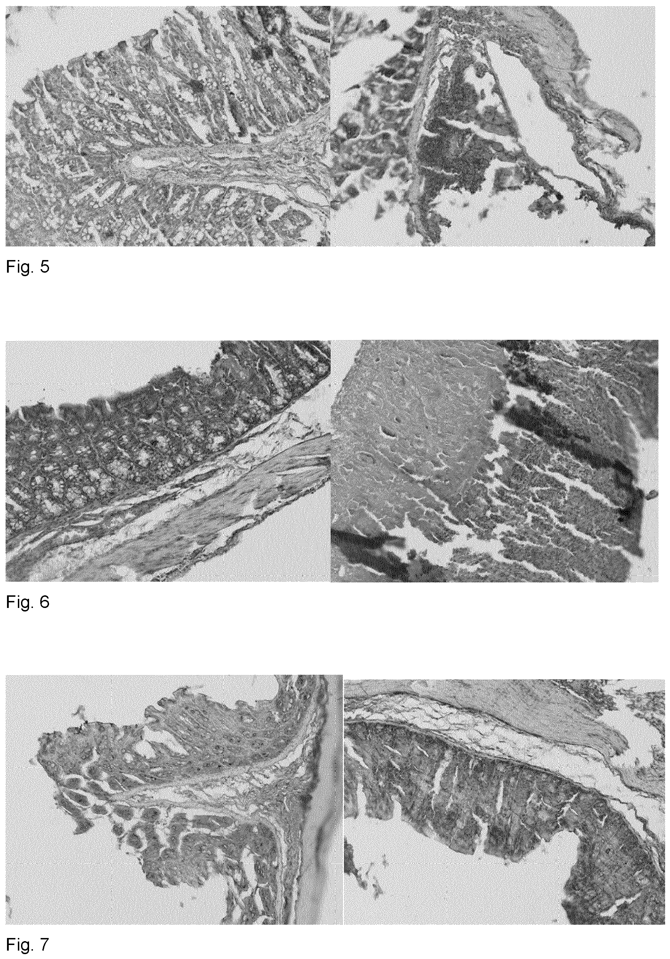

Fig. 5: 2o - minor catarrhal inflammation, isolated erosions (proximal and distal parts - left to right). This sample has been taken from experimental (treatment) group
Fig. 6: 3o - mild diffuse catarrhal inflammation, a large focus of fibrinous-haemorrhagic inflammation (proximal and distal parts - from left to right). This sample has been taken from experimental (treatment) group.
Fig. 7: 4o - mild catarrhal inflammation, slight hyperplasia of goblet cells (proximal and distal parts - from left to right). Sample from experimental (treatment) group.

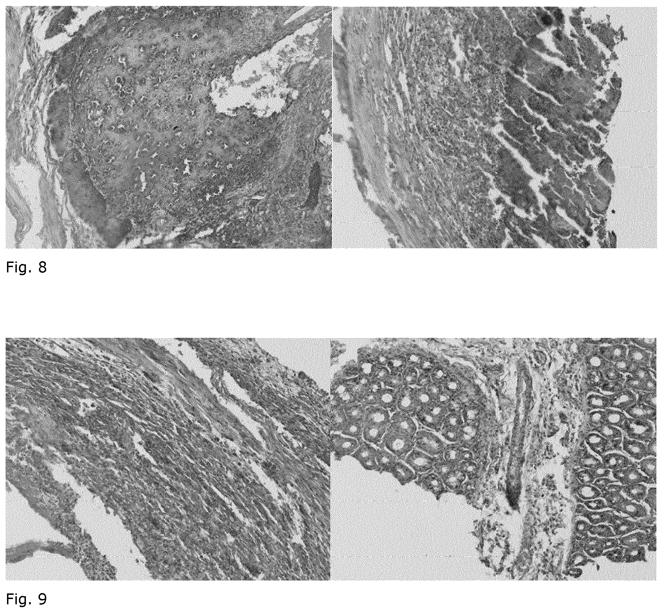

Fig. 8: 1κ - Numerous widespread ulcers extending to the serosal layer, massive neutrophilic infiltration of the mucosa, crypt abscesses (proximal and distal parts - from left to right). This sample has been taken from control (saline) group.
Fig. 9: 4κ - diffuse neutrophilic infiltration extending to the muscular layer of the mucosa, submucosal fibrosis, and muscularis propria (proximal and distal parts - from left to right). This sample has been taken from control (saline) group.

### DETAILED DESCRIPTION

In the following detailed description, preferred embodiments are described in detail to enable practice the invention. Although the invention is described with reference to these specific preferred embodiments, it will be understood that the invention is not limited to theses preferred embodiments. To the contrary, the invention includes numerous alternatives, modifications and equivalents as will become apparent from consideration of the following detailed description.

In a first aspect, the object of the invention is achieved by providing a pharmaceutical composition comprising: hyaluronic acid, sodium dihydrophosphate monohydrate, sodium phosphate anhydrous, sodium chloride and deionised water, characterized in that it further comprises chondroitin sulphate and mucoadhesive biopolymer.

The pharmaceutical composition according to invention is intended for administration of a single application dose in the bladder or rectum via a urethral or rectal catheter, or rectal applicator. Composition has a protective effect on the bladder, rectal and colon mucosa and at the same time has a tissue repair and regeneration effect.

Hyaluronic acid is a component of the protective layer of the mucous membranes of the human body, including the rectal and colon mucosa. However, in addition to hyaluronic acid, the protective layer of the intestines and other hollow organs (bladder, esophagus) includes chondroitin sulphate. Thus, to increase efficiency, inventors added chondroitin sulphate to the composition. Also, the effectiveness of a composition depends on its exposure. Contact with the epithelium should be at least half an hour. However, one of the symptoms of ulcerative colitis is diarrhea (frequent bowel movements). The urge to defecate increases with rectal administration of the solution. Therefore, it is necessary to add to the composition an agent that enhances adhesion (attachment force) to the epithelium. i.e. mucoadhesive agent.

The mucoadhesive biopolymer enhances the mucoadhesive effect of the product.

Invention composition provides the protection and restoration of the epithelium of the bladder or rectum.

HA assures the mucoprotective effects, ability to promote healing via epithelial restitution in the lumen and bioadhesive effects that prolongs the retention of active substances at the site of inflammation and thus increases the contact time with the intestinal epithelium and consequently the absorption and effect of the active substance.

The mucoadhesive polymer enhances the adhesion of drugs to the mucosa of the bladder or intestines and, even with insufficient exposure, allows the composition to attach to the mucosa.

In one embodiment of the invention, the pharmaceutical composition comprises hyaluronic acid in the range of from 0.8 mg to 16.0 mg, sodium dihydrophosphate monohydrate in the range of from 0.3 mg to 0.6 mg, sodium phosphate anhydrous in the range of from 0.5 mg to 4 mg, sodium chloride 8.5 mg, chondroitin sulphate in the range of from 2.0 to 16.0 mg, mucoadhesive biopolymer in the range of from 5.0 mg to 20.0 mg and deionised water up to 1 ml.

In one preferred embodiment of the present invention, the pharmaceutical composition comprises in each 1 ml: hyaluronic acid 0.8 mg, sodium dihydrophosphate monohydrate 0.45 mg, sodium phosphate anhydrous 2.0 mg, sodium chloride 8.5 mg, chondroitin sulphate 2.0 mg, mucoadhesive biopolymer 10.0 mg and deionised water up to 1ml.

In one embodiment of the invention, the pharmaceutical composition is for use in the prevention, treatment, or alleviation of ulcerative colitis.

The composition is administered as rectal instillation once a day or every other day up to 30 procedures in a single application dose of 20 ml to 50 ml via rectal catheter or rectal applicator for a period from 30 minutes to at least 120 minutes.

In another embodiment of the invention, the pharmaceutical composition is for use in the prevention, treatment, or alleviation of Interstitial Cystitis/Bladder Pain Syndrome (IC/BPS).

The composition is administered as weekly instillations for 4 weeks, followed by 2 instillations every 2 weeks (at 6 and 8 week) and 2 instillations monthly in a single application dose of 1 ml to 50 ml via urethral catheter for a period from 30 minutes to at least 120 minutes.

Thus, the invention is a composition for use in the prevention, treatment or alleviation of interstitial cystitis or ulcerative colitis by the method of introduction through a catheter into the bladder (bladder instillation) or rectum (rectal instillation) as part of the components of the glycosaminoglycan layer: hyaluronic acid of various molecular weights; chondroitin sulphate of various molecular weights and mucoadhesive biopolymer (one or a combination thereof).

Mucoadhesive polymer can be carrageenan, gelatine, gums, hydroxypropylmethylcellulose (HPMC), and methylcellulose (MC), hydroxypropylcellulose (HPC), ethylcellulose (EC), polycarbophil, cyanoacrylate, hyaluronic acid, poly(vinyl alcohol) (PVA), polyacrylic acid (PAA) and poly(hydroxyalkyl methacrylate).

Preferably, the mucoadhesive polymer is selected from the group: sodium alginate, chitosan, hydroxyethyl cellulose (HEC), polloxamer (thermoplastic polymer), polyethylene glycol, polyvinylpyrrolidone, polyacrylic acid, polyvinyl alcohol (PVA), sodium carboxymethylcellulose (NaCMC) or combinations thereof.

Most preferably, the mucoadhesive polymer is sodium alginate, since in addition to the mucoadhesive effect it has a regenerating effect.

Preferably the hyaluronic acid is in the form of sodium hyaluronate.

Inventors add sodium alginate to the composition with hyaluronic acid and chondroitin sulphate, because it has not only a mucoadhesive, but also a regenerating effect.

In another embodiment, the composition comprises further a mixture of comenic acid, gluconic, and 2-ketogluconic acid, which have antibacterial, antioxidant and regenerative effects.

In a further aspect, the present invention provides a medical device comprising the pharmaceutical composition in a package with leaflet.

The object of the invention is solved by the fact that patients after clinical and laboratory examination: rectoromanoscopy, anoscopy, fibrocolonoscopy with biopsy, faecal calprotectin analysis, can be prescribed pathogenetic therapy with 5-aminosalicylic acid preparations, in addition to which a rectal injection of the pharmaceutical composition according to the invention, heated to 35-37 °C into the rectum using a microclysis or syringe with a flexible disposable rectal catheter with a length corresponding to the middle of the localisation of the ulcerative lesion or rectal applicator, 20 ml to 50 ml once a day every other day, mainly before bedtime; after the manipulation the patient lies on the left side with bent lower limbs in the hip joints in a horizontal position for at least 2 hours, the course of 30 procedures. The introduction of warm (temperature about 35-38 ° C) drug at a given depth in the position on the left side, contributes to improved bioavailability and better tropism of therapy. This promotes relaxation of the colon, has an antispasmodic effect, and stimulates intestinal motility better than cold or cool microclysms. Warm microclysms dissolved the faeces better and facilitated better absorption of the therapeutic agent. The study of faecal calprotectin shall be before treatment, 30 and 60 days after the start of the product, and if the level of faecal calprotectin decreases less than 50 µg/g, the introduction of the product can be discontinued.

The technical result obtained by using the claimed composition consists in reducing the healing time of erosions and ulcers of the mucosa of the rectum and sigmoid colon, due to the creation of favourable conditions for regeneration in reducing the incidence of recurrences after treatment, as well as reducing the incidence of complications of the underlying disease.

Effective restoration of the mucosa of the terminal section of the large intestine and suppression of purulent-inflammatory and erosive-ulcerous lesions is achieved due to the enveloping and regenerating properties of the components included in the product: hyaluronic acid, sodium dihydrophosphate monohydrate, sodium phosphate anhydrous, sodium chloride, chondroitin sulphate, mucoadhesive biopolymer and deionised water. The hyaluronic acid reduces the permeability of the mucous membrane, accelerates epithelialisation, and stops purulent inflammatory phenomena in erosions and ulcers of the terminal section of the large intestine. At restoration of the mucous layer the reverse diffusion of hydrogen ions decreases, which contributes to the retention of histamine and pepsin, the management of mucosal oedema and reduction of capillary permeability of the intestinal mucosa. This contributes to the achievement of the technical result.

The pharmaceutical composition according to invention for use in the prevention, treatment, or alleviation of patients with inflammatory diseases of the terminal parts of the large intestine, contributes to the reduction of pain syndrome, normalisation of stool quality, reduction of the frequency of defecation acts to the number of normal, improvement of the microflora of the large intestine, reduces the period of treatment of patients in hospital, helps to avoid complications.

The general condition of the patient and duration of conservative therapy can be assessed on the basis of mucosal biopsy of the large intestine before treatment, after 1 month and after 2 months, which allows to adequately assess the dynamics of the treatment process and timely respond to possible pathological changes. Analysis of the results of faecal calprotectin study before treatment, 30 and 60 days after the beginning of the product administration allows to determine the required number of injections (doses) of the drug. Decrease of faecal calprotectin level below 50 µg/g indicates the disease transition to the remission stage and consideration of the need to continue treatment or reduce the dosage of drug administration.

The composition according to the invention has regenerating properties due to improvement of tissue hydrodynamics, enhancement of the process of migration and proliferation of cells, as well as contributing to the complete suppression of purulent-inflammatory and erosive-ulcerous lesions due to the binding of hyaluronic acid with aggrecan monomers in the presence of binding protein, due to which large negatively charged aggregates absorbing water are formed in collagen and elastic fibres. These aggregates are responsible for the elasticity, integrity and rapid regeneration of these fibres.

Claimed pharmaceutical composition is a sterile viscoelastic protector for the rectal mucosa. Characteristics of the product: sterility; absence of harmful substances; viscosity; transparency and solution without sediment.

On the basis of experimental and clinical studies inventors proved the necessity of adequate impact on the mucosa of the rectum and sigmoid colon, to stop the infectious-inflammatory process in all patients with this disease. The product significantly reduces the amount of purulent discharge from petechial and erosive rashes on the mucosal surface of the distal colon, reduces oedema and hyperaemia, triggers the regeneration process. This, in turn, reduces the time of wound healing, leads to a decrease in the incidence of complications and recurrence of the disease.

### EXAMPLES

The invention will be described below in detail with reference to examples but the invention is not limited in any way by these examples, as long as it does not exceed the scope and spirit of the present invention.

### Example 1

Pharmaceutical composition comprises in each 1 ml:
hyaluronic acid 0.8 mg,
sodium dihydrophosphate monohydrate 0.45 mg,
sodium phosphate anhydrous 2.0 mg
sodium chloride 8.5 mg
chondroitin sulphate 2.0 mg
chitosan 10.0 mg
deionised water up to 1ml.

### Example 2

Pharmaceutical composition comprises in each 1 ml:
sodium hyaluronate 1.8 mg,
sodium dihydrophosphate monohydrate 0.45 mg,
sodium phosphate anhydrous 2.0 mg,
sodium chloride 8.5 mg,
chondroitin sulphate 5.0 mg,
sodium alginate 10.0 mg
deionised water up to 1ml.

### Example 3

Pharmaceutical composition comprises in each 1 ml:
hyaluronic acid 3.0 mg,
sodium dihydrophosphate monohydrate 0.45 mg,
sodium phosphate anhydrous 2.0 mg,
sodium chloride 8.5 mg,
chondroitin sulphate 3.0 mg,
hydroxyethyl cellulose 10.0 mg
deionised water up to 1ml.

### Example 4

Pharmaceutical composition comprises in each 1 ml:
hyaluronic acid 2.0 mg,
sodium dihydrophosphate monohydrate 0.45 mg,
sodium phosphate anhydrous 2.0 mg,
sodium chloride 8.5 mg,
chondroitin sulphate 10.0 mg,
PVP 20.0 mg
deionised water up to 1ml.

### Example 5

Pharmaceutical composition comprises in each 1 ml:
hyaluronic acid 8.0 mg,
sodium dihydrophosphate monohydrate 0.45 mg,
sodium phosphate anhydrous 2.0 mg,
sodium chloride 8.5 mg,
chondroitin sulphate 16.0 mg,
sodium alginate 15.0 mg
deionised water up to 1ml.

### Example 6

Pharmaceutical composition comprises in each 1 ml:
hyaluronic acid 16.0 mg,
sodium dihydrophosphate monohydrate 0.45 mg,
sodium phosphate anhydrous 2.0 mg,
sodium chloride 8.5 mg,
chondroitin sulphate 8.0 mg,
sodium alginate 15.0 mg
deionised water up to 1ml.

The pharmaceutical composition is to be applied in volumes from 20 to 50 ml.

### Materials and methods

### In vivo animal studies

Ulcerative colitis was induced after 24 hours of fasting (with access to water) by rectally administering 1 ml of 6% acetic acid to a depth of 8 cm, with a 1-day interval between administrations. Lesions in the animals occurred within the first hour of acetic acid administration and resolved within 3-5 days. Clinical manifestations, such as bloody diarrhea and weight loss, were observed following the introduction of acetic acid into the intestinal cavity. Therapy commenced on the subsequent day after the second acetic acid administration: the experimental group received a hyaluronic acid-based protector (sodium hyaluronate, chondroitin sulphate, sodium alginate) while the control group received a saline solution. The drugs were administered rectally in a volume of 1 ml.

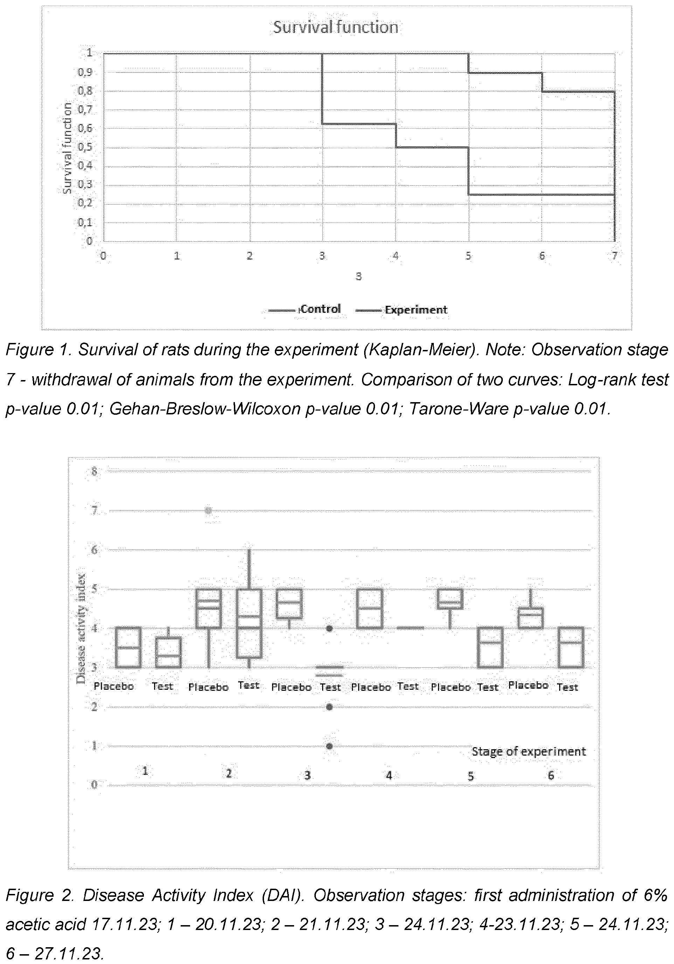

Survival of rats during the experiment (Kaplan-Meier) is shown on Fig.1. Survival is significantly higher in experimental group (treatment with rectal instillations of hyaluronic acid, chondroitin sulphate and sodium alginate).

Clinical status was assessed using scores on the modified Disease Activity Index (DAI) scale adapted for rats (Fig.2 and Table 1).

**Table 1 Disease Activity Index**

| **Symptom** | **Score** | **Description** |
|---|---|---|
| Loss of body weight | 0 | 0% |
| | 1 | 1-5% |
| | 2 | 6-10% |
| | 3 | 11-20% |
| | 4 | More 20% |
| Stool consistency | 0 | Normal |
| | 1 | Normal |
| | 2 | Loose stools |
| | 3 | Loose stools |
| | 4 | Diarrhoea |
| Rectal Bleeding | 0 | Normal |
| | 1 | Occult blood + |
| | 2 | Occult blood ++ |
| | 3 | Occult blood +++ |
| | 4 | Gross bleeding |

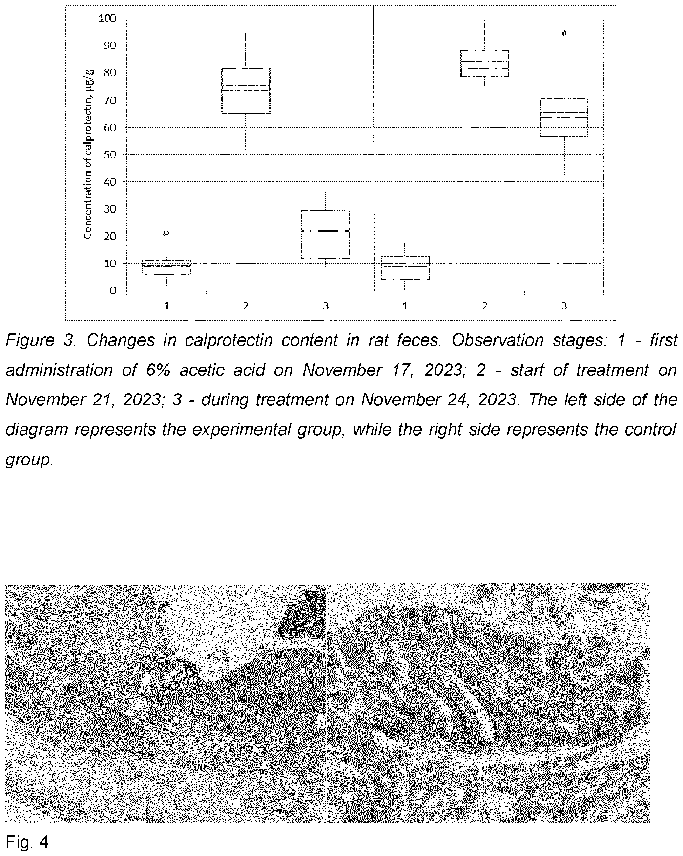

Changes in calprotectin content in rat feces are shown on Fig.3. Calprotectin level is significantly higher in saline (control) group (65 mcg/g). The level of calprotectin in experimental group (sodium hyaluronate, chondroitin sulphate, sodium alginate) is 22 mcg/g.

The Disease Activity Index is also higher in control (saline) group: 4.8 in comparison with 3.6 in experimental (hyaluronic acid, chondroitin sulphate, sodium alginate) group.

### Results of histological examination of rat intestinal samples

In the experimental group, mild catarrhal inflammation with slight hyperplasia of goblet cells was observed. In the control group, there were pronounced ulcerative-necrotic processes, diffuse and focal purulent inflammation in the mucous membrane, and fibrosis. This shows, that composition (hyaluronic acid, chondroitin sulphate, sodium alginate) is more effective in comparison with placebo.

## Claims

1. Pharmaceutical composition comprising:
hyaluronic acid, sodium dihydrophosphate monohydrate, sodium phosphate anhydrous, sodium chloride and deionised water,
**characterized in that** it further comprises chondroitin sulphate and mucoadhesive biopolymer.

2. Pharmaceutical composition according to claim 1 **characterized in that** it comprises hyaluronic acid in the range of from 0.8 mg to 16.0 mg, sodium dihydrophosphate monohydrate in the range of from 0.3 mg to 0.6 mg, sodium phosphate anhydrous in the range of from 0.5 mg to 4.0 mg, sodium chloride 8.5 mg, chondroitin sulphate in the range of from 2.0 to 16.0 mg, mucoadhesive biopolymer in the range of from 5.0 to 20.0 mg and deionised water up to 1 ml.

3. Pharmaceutical composition according to claim 1 or 2 **characterized in that** it comprises in each 1 ml: hyaluronic acid 0.8 mg, sodium dihydrophosphate monohydrate 0.45 mg, sodium phosphate anhydrous 2.0 mg, sodium chloride 8.5 mg, chondroitin sulphate 2.0 mg, mucoadhesive biopolymer 10.0 mg and deionised water up to 1 ml.

4. Pharmaceutical composition according to any of the claims 1 to 3, **characterized in that** the mucoadhesive biopolymer is selected from the group: sodium alginate; chitosan; hydroxyethylcellulose; polloxamer (thermoplastic polymer); polyethylene glycol; polyvinylpyrrolidone; polyacrylic acid; polyvinyl alcohol; sodium carboxymethylcellulose; or combinations thereof.

5. Pharmaceutical composition according to claim 4, **characterized in that** the mucoadhesive biopolymer is sodium alginate.

6. Pharmaceutical composition according to any of the preceding claims, **characterized in that** hyaluronic acid is in the form of sodium hyaluronate.

7. Pharmaceutical composition according to any of the preceding claims, **characterized in that** the composition comprises further a mixture of comenic acid, gluconic, and 2-ketogluconic acid.

8. Pharmaceutical composition according to any of the claims 1 to 7, **characterized in that** the composition is intended for administration of a single application dose in the bladder or rectum via urethral or rectal catheter or rectal applicator.

9. Pharmaceutical composition according to any of the claims 1 to 7 for use in the prevention, treatment or alleviation of ulcerative colitis.

10. Pharmaceutical composition according to claim 9, **characterized in that** the composition is administered as rectal instillation once a day or every other day up to 30 procedures in a single application dose of 20 ml to 50 ml via rectal catheter or rectal applicator and keep it in rectum for a period from 30 minutes to at least 120 minutes.

11. Pharmaceutical composition according to any of the claims 1 to 7 for use in the prevention, treatment, or alleviation of Interstitial Cystitis/Bladder Pain Syndrome.

12. Pharmaceutical composition according to claim 11, **characterized in that** the composition is administered as weekly bladder instillations for 4 weeks, followed by 2 instillations every 2 weeks (at 6 and 8 week) and 2 instillations monthly in a single application dose of 1 ml to 50 ml via urethral catheter and keeping the composition in the bladder for a period from 30 minutes to at least 120 minutes

13. Medical device comprising the pharmaceutical composition according to claim 1 to 7 in a package with leaflet.
